# EUROPEAN PATENT APPLICATION

(11) **EP 2 842 527 A1**
(43) Date of publication of application: **04.03.2015**
(21) Application number: 13004208.8
(22) Date of filing: 26.08.2013
(51) Int. Cl.: A61F 5/01, A61H 3/00, A61F 2/68, B25J 9/00

(54) **Wearable posture assisting device**

(71) Applicant: ETH Zurich, 8092 Zurich (CH)
(72) Inventor: Iida, Fumiya, 8057 Zürich (CH); Anastasiades, Bryan, 8704 Herrliberg (CH); Gunura, Keith, 8600 Dübendorf (CH)

(57) **Abstract**

The invention is a wearable mechatronic posture assisting device comprising an upper support (10) for connecting to the thigh of a person, a lower support (20) for connecting to the shank of the person and a joint (28) pivotably connecting both supports (10, 20), and a damper (30) with connectors (31 a, 32a) for pivotably connecting the damper (30) to the supports (10, 20). The damper (30) is designed to linearly damp the pivot of the joint (28). The damper (30) comprises a control means allowing a locking and a drive state (101, 103) of the damper (30).

## Description

### Technical field

The invention is related to a mechatronic posture assisting device. Such a device can be worn engaging a damper and/or brake function in order to assist the user with his body weight, limb weight and/or even objects being carried. The device usually takes the load off the users muscles in any posture.

### Background of the invention

Assisting devices for different purposes and according to different categories are well known. In US 4 138 156 A an apparatus is disclosed supporting the weight of a person's body comprising a unit for attachment for each leg. Each unit includes a lower bar having an upper section and a lower section which is longitudinally extendable from the upper section. An upper bar is hingeably connected to the upper end of the lower bar. A brace member, pivotable from the upper bar, is selectively positionable between the upper bar and the lower section of the lower bar. The apparatus puts the weight of the person or a part of it onto the ground on which the lower section of the lower part rests. This apparatus has some constraints which to overcome is one aspect of the instant invention. Since the apparatus has no active elements, no brakes and no damper, no automated control is possible. Furthermore, an advantageous apparatus should not necessarily direct the force just to the ground. It is also disadvantageous when only a single very limited number of postures are supported.

In EP 2 380 529 A1 a knee orthosis and methods of controlling the knee orthosis are disclosed comprising an actuator between the thigh and the shank. The device controls damping based on sensory feedback. The behaviour of the actuator unit depends on measured knee moment and knee angle. However, this knee orthosis has no posture support and is not designed to support the body weight and has no brake. The knee othosis is designed to be used for the rehabilitation of a knee only. In US2009/0184476 A1 a orthopaedic knee joint brace is disclosed having an anatomically shaped thigh and shank support. Upper or lower cuff are made of a reinforced plastic with a shape corresponding to thigh and shank region. However, this means has no posture support and is also designed to be used for the rehabilitation of a knee only. A similar device is disclosed in US 2009/182 254 A1.

In US 2012/259 431 A1 a terrain adaptive power joint orthosis is disclosed which is designed as a powered human augmentation device assisting a person walking on a surface comprising a powered actuator for supplying a linear spring component and a damping component. A controller estimates gait cycle and adjusts to slope and stairs. This device, however, has no posture or active walking support. Additionally, the powered actuator for support applies external forces on the leg and knee in particular. Similar electrical power actuator devices are known. These devices, however, require an external power source which limits the use and implies an additional heavy weight to be carried.

A device for supporting a human body in various positions is disclosed in US 2009/058151 A1 providing an ergonomic chair which is fixed to the ground or on sliding rails. The device, however, is not wearable and walking with the device not possible.

Trousers associated with a ground engaging improved support for seat engagement by wearer are disclosed in US 3 538 512 A. These trousers are designed as a combination of garment including trousers for a wearer having a pair of legs joining with the waist, each of the legs having a means there along for receiving an elongated rod means extending. The rod is dislocated from the bottom half during walking, whereas the rod slides into the lower half to create a rigid vertical structure when seating. Again, these trousers redirect force to ground thereby providing limited possibilities for posture support and no damping at all.

### Description of the invention

The invention has the object to provide a posture assisting device with extended possibilities for posture support, which device should be wearable also during walking. Additionally, the device should include at least dampers and the device should be controlled to support the predetermined posture of the possible postures. Furthermore, a device should be provided without any extended electrical power consumption or like, at least to remove the need for actively powered actuators and use a natural force, gravity, to drive the system for actuation. A holding force that will remain engaged passively using only gravity and the body mass for activation will reduce the energy consumption. The user shall be the driver of the device and not beassisted in movement or actions except only when having to maintain a posture for some desired period of time.

The object of the invention is solved by the posture assisting device according to claim 1. The posture assisting device comprises an upper support for connecting to the thigh of a person and a lower support for connecting to the shank of said person. A joint, which joint is preferably designed as an articulated joint, connects the upper support and the lower support, and a damping means with two ends - an upper end and a lower end - and an upper connector for pivotably connecting the upper end to the upper support, preferably at or adjacent to the joint or at the location were the joint is connected to the upper support with a lower connector for pivotably connecting the lower end to the lower support, preferably at the middle third of the lower support, or at the third quarter - of the top. The damping means are designed to linearly damp the drive between its two ends, thereby damping the pivot of said joint. The damping means comprise a control means, said control means at least having a locking state locking the drive of the damper and a drive state which may be designed as an "open" state of a valve, allowing the drive of the damper which is a strong but light damping unit which is able to passively engage large holding forces. The properties of the damping unit can be controlled either manually or with an electric motor.

The support structure is optimized for weight and size and can ultimately be implemented into the wearer's clothes. The cost effective design allows providing devices which are affordable to a broader target group than current exoskeletons and other posture supports.

The invention as a wearable mechatronic posture assisting device that, when worn, can engage a damper and brake function, assists the user with his body weight, limb weight or even objects being carried. The device takes the load off the users muscles in any posture. The device herein called "Chairless Chair" is one example of such a posture support for the lower limbs. It consists of a support structure and a damper. The support structure itself consists of an upper part which is strapped to the upper leg and a lower part which is strapped to the heel of the shoes of the user. One essential advantage of this mechanism over most of the currently available solutions is the low energy consumption. The locking and damping can be done passively. Together with the very high force to weight ratio this allows decreasing the overall weight of the device and therefore the movability of the user wearing this device. The cost effective design allows providing devices which are affordable to a broader target group than current exoskeletons and other posture supports.

With this device being widely available the risk of musculoskeletal disorders (MSD) and therefore the loss of productivity due to sick days of the employees can be reduced. The mechanism can also be used in other fields like for exercising in rehabilitation or for home trainers.

Advantageous embodiments of the invention are described in the dependent claims.

In an advantageous embodiment of the invention a connecting rod at the lower support connects the lower connector of the damper to the lower support so that a force conducted from the upper support onto the upper end of said damper and from the damper onto the lower support is essentially directed onto the lower support to the forward direction of the person.

It is also advantageous when the lower support comprises additional connecting means for connecting the lower part of said lower support to the heel of the person or the heel of the shoe of the person, redirecting the force conducted from the upper support onto the upper end of said damper and from the damper onto the lower support is redirected forward onto the heel of the person or the heel of the shoe of the person.

According to one aspect of the invention, the damping means comprise a liquid-type damper or a viscous type damper. The control means include a valve. Preferably, the control means additionally allow one or more states with a lower movement, corresponding to a higher drag, than the drive state (open state). The control means could allow to continuously control the movement between said locking state and said drive state (open state).

According to a basic configuration of the invention, the control means comprise a manually driven lever to control the valve. In a more advanced configuration the said control means comprise an electrical driven valve. The skilled person will note that the electrical power necessary to change the state of the valve cannot be compared with the power necessary to drive an actuator to change the posture as provided by some device according to the above-discussed prior art.

The posture assisting device can have laces for connecting the upper support to the thigh of the person and/or for connecting the lower support to the shank of the person, preferably by means of hook-and-loop fasteners. It should be noted that the upper support as well as the lower support can be formed so that the back part of the thigh or the shank comfortably fit to the respective support. In an advantageous embodiment of the invention the upper support and/or the lower support are adjustable in respect of the length as to fit comfortably to persons with different length of the thigh and the shank, respectively.

The aforementioned elements as well as those claimed and described in the following exemplary embodiments, to be used according to the invention, are not subject to any particular conditions by way of exclusion in terms of their size, shape, use of material and technical design, with the result that the selection criteria known in the respective field of application can be used without restrictions. It should be noted that all means used to implement the invention are not limited to a particular design. The mechanism of the invention is not limited to the lower limbs but can also be used for arms, shoulders and back support.

The skilled person will note that the usage of the posture assisting device according to the present invention is not limited to the usage for workers and for other persons to be assisted during their work, i.e. at a band-conveyor. Moreover, the posture assisting device can be used supporting the rehabilitation of injured persons or even for assistance of persons having disseminated sclerosis. One main usage can be the assistance for recreational activities, i.e. fishing and hunting.

### Brief description of the drawings

Examples of the posture assisting device will hence forth be described in more detail by reference to the drawings, wherein are shown:
- Figure 1: a side view of a preferred embodiment of the invention worn by a person,
- Figure 2a: a perspective view of the embodiment according to figure 1,
- Figure 2b: a perspective view according to figure 2a from another angle,

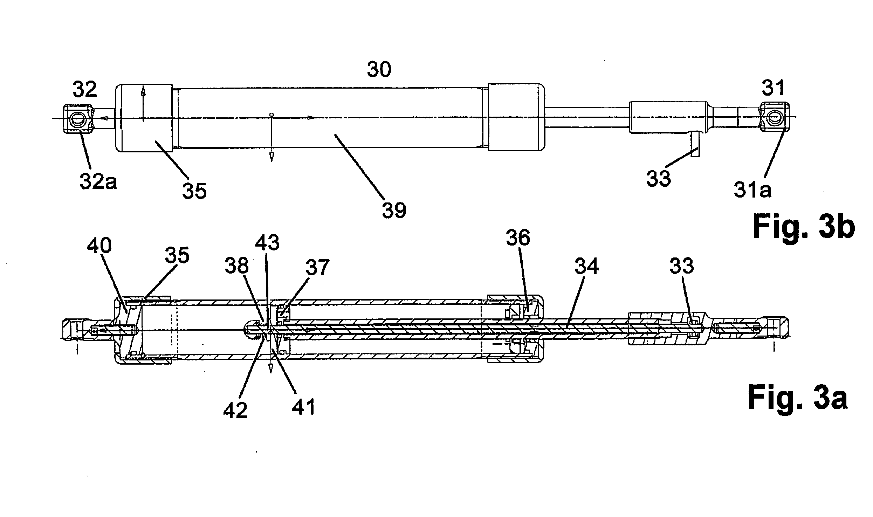

- Figure 3a: a view onto the main elements of the damping means substructure according to figures 1 and 2a, 2b;
- Figure 3b: a view onto the damping means according to figures 3a;
- Figure 4: a view onto the valve according to a preferred embodiment in different positions with the functions closed, damped and open,
- Figure 5: a side view according to figure 4 with the functions closed and open.

### Embodiments for carrying out the invention

The embodiment described hereby is one preferred embodiment of the invention called "Chairless Chair".

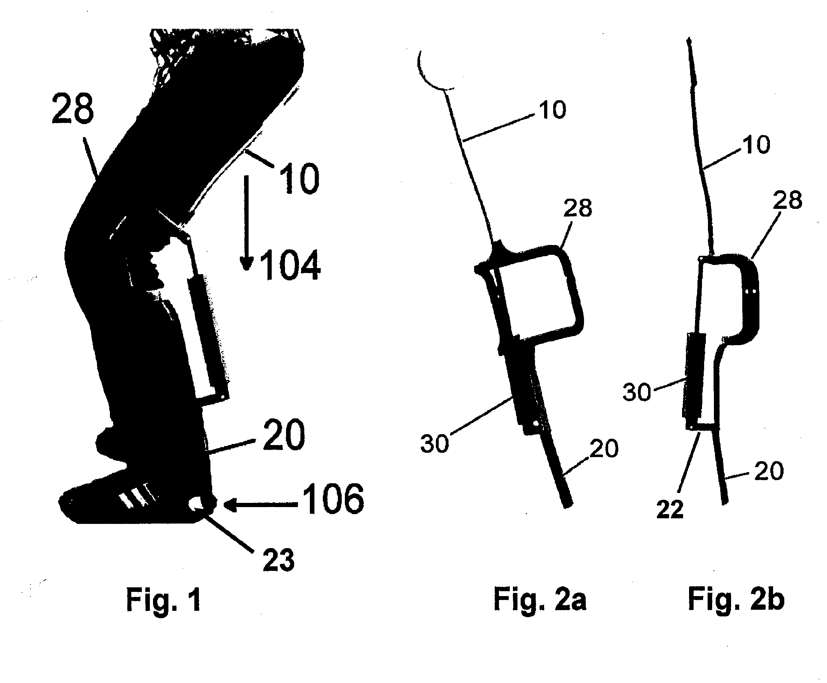

The "Chairless Chair" comprises a support structure with an upper support 10 for connecting to the thigh of a person, a lower support 20 for connecting to the shank of the person and damping means 30. A joint 28 connects the upper support 10 and the lower support 20. The support structure with the upper support 10 and the lower support 20 is ergonomically shaped to provide a good fit to the legs and is designed to ensure a comfortable feeling. The strapping is not shown in figure 1. The strapping comprises soft padding which allows a firm fit of the support structure to the leg with a well-distributed pressure.

The joint 28 is - according to the described embodiment - designed as an articulated joint connecting the upper support and the lower support. It is a normal pin hole joint or - alternatively - an auto-adjustable joint that follows the natural movement of the center of rotation of the knee joint. In this example the force of the body weight gets absorbed by the support structure and the damper and then redirected to the heel connector.

The damping means 30 comprise a hydraulic variable damper. It can vary the resistance from very low damping up to very high damping and ultimately brake. The damper has two ends - an upper end 31 and a lower end 32 - and an upper connector 31 a for pivotably connecting the upper end 31 to the upper support 10, preferably at or adjacent to the joint or at the location were the joint 28 is connected to the upper support 10 with a lower connector 32a for pivotably connecting the lower end 32 to the lower support 20, preferably at the middle third of the lower support 20, or at the third quarter - from the top. The damping means 30 are designed to linearly damp the drive between its two ends 31 and 32, thereby damping the pivot of said joint 28. The damping means 30 comprise a control means, said control means at least having a locking state locking the drive of the damper 30 and a drive state which may be designed as an "open" state of two disc valve, allowing the drive of the damping means 30. The manual handle 33 of the damper is used to control the relative angle between the discs of the rotary two disc valve. It can be replaced by a motor, i.e. an electrical motor. An inner shaft 34 is connected to the disc at the end of the piston with opening 37 and to the manual handle. The inner shaft 34 translates the movement of the handle to the disc 41. A cover nut 35 secures the guider and the tube sealing 40 on the hydraulic tube 39. The linear guider assembly 36 restricts the movement of the piston to the axial direction and seals of the top end of the hydraulic tube 39. The piston with opening is considered as the core element of the rotary two disc valve. It absorbs the damping and braking force, respectively from the host device. The disc 41 with opening is the second element of the rotary two disc valves. Its relative turning angle to the piston defines the amount of damping and, when the disc 41 closes the flow of the hydraulic fluid, the brake function, respectively. A release valve 38 is installed to allow emergency push open of the brake when locked. The hydraulic tube 39 contains the hydraulic fluid and restricts the piston to a linear movement. The tube sealing 40 seals the bottom part of the hydraulic tube 39.

The force redirection according to the embodiment called "Chairless Chair" is shown by the arrows 104 and106 in figure 1. The body weight 104 gets absorbed by the upper support 10, the damping means 30 when in the operation mode as a brake with the valve closed, the lower support 20 and then gets redirected to the heel of the person or the person's shoe according to the arrow 106. Because of the shape of the support structure and the position of the straps the direction of the force changes from the vertical direction (down) to mainly horizontal direction (forward). The mechanization of the redirection is supported by the connecting means 22 at the lower support 20 connecting the lower end of the damper 30 to the lower support 20. The connecting means 22 are arranged on the lower support such that a component of the stress is redirected from the support to a part of the body or equipment, e.g. the heel of the shoes. Means 23 for connecting the lower part of said lower support 20 to the heel of the person or the heel of the shoe of the person are provided therefore.

### Valve operation principle

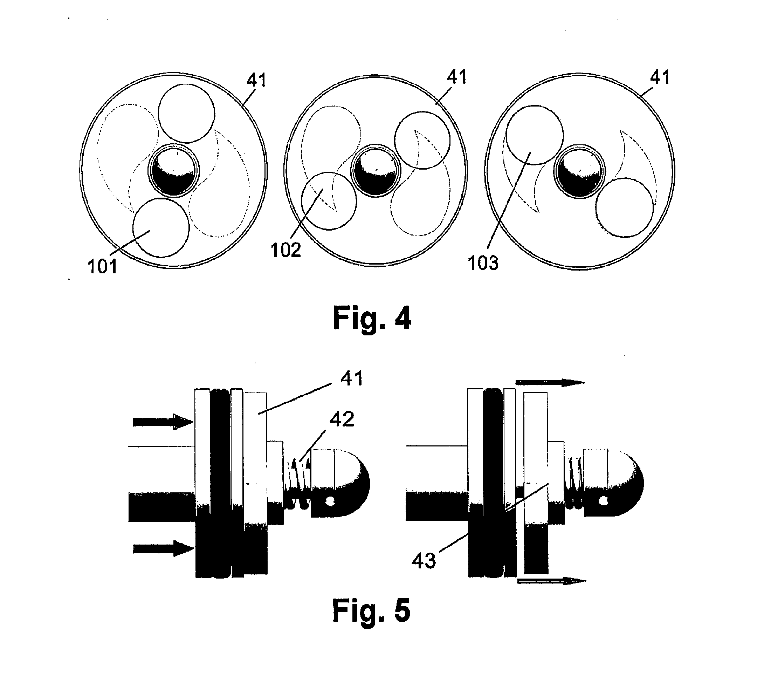

The operation principle of the two disc valve is illustrated in figure 4 and described here thereafter.

When the two discs of the valve are perfectly overlapped - situation 101 - then the valve is closed and the damper acts as a brake. If the holes in the two discs are perfectly aligned then the valve is open - situation 103. The amount of resistance to the flow of the working fluid depends and the effective opening area of the orifice. The effective orifice area can be changed by rotating one disc relative to the other - situation 102. The damping can therefore be continuously changed from very low damping 103 to very high damping and brake 101.

### Safety Mechanism

To ensure possibility to unlock the damper even when the brake is engaged a safety mechanism was included into the valve. The working principle of the safety mechanism is shown in figure 5. The rotatable disc 41 is in lock position, the brake is engaged. When needed the piston can be pushed in the opposite direction (extension of damper). The spring 42 acts as compliance and allows the disc 41 to travel away from the piston due to the fluid stream pressing against disc 41. This leads to a small gap between the two discs and therefore the valve is no longer closed. A small catch 43 ensures that the disc is still aligned in its relative rotation. So when the pressure from extending the damper drops the disc 41 is pushed back onto the piston disc by spring 42.

### List of reference signs

- 10: upper support
- 20: lower support
- 22: connecting means
- 23: means for connecting the lower part of said lower support to the heel
- 28: joint
- 30: damping means
- 31: upper end of the damper
- 31a: upper connector
- 32: lower end of the damper
- 32a: lower connector
- 33: manual handle
- 34: inner shaft
- 35: cover nut
- 36: linear guider assembly
- 37: piston with opening
- 38: release valve
- 39: hydraulic tube
- 40: tube sealing
- 41: disc with opening
- 42: spring
- 43: small catch
- 101: situation of closed valve
- 102: situation of partly opened valve
- 103: situation of fully opened valve
- 104: gravitational force/body weight
- 106: redirected force

## Claims

1. A posture assisting device comprising
- an upper support (10) for connecting to the thigh of a person,
- a lower support (20) for connecting to the shank of said person,
- a joint (28) pivotably connecting said upper support (10) and said lower support (20),
- a damping means (30) having two ends (31, 32) and comprising an upper connector (31 a) for pivotably connecting the upper end (31) to the upper support (10), preferably at or adjacent to the joint (28) and further comprising a lower connector (32) for pivotably connecting the lower end (32) to the lower support (20), preferably at the middle third of the lower support (20),
**characterized in that**
- said damping means (30) are designed to linearly damp the drive between its two ends (31, 32), thereby damping the pivot of said joint (28),
- said damping means (30) comprises a control means, said control means at least having a locking state (101) locking the drive of the damping means (30), and a drive state (103) allowing the drive of the damping means (30).

2. The posture assisting device according to claim 1, **characterized by** a connecting means (22) at the lower support (20) for connecting the lower end of the damping means (30) to the lower support (20) so that a force conducted from the upper support (10) onto the upper end (31) of said damping means (30) is directed from the lower end (32) of said damping means (30) onto the lower support (20).

3. The posture assisting device according to claim 1 or 2, **characterized in that** said lower support comprises means (23) for connecting the lower part of said lower support (20) to the heel of the person or the heel of the shoe of the person, redirecting the force conducted from the upper support (10) onto the upper end (31) of said damping means (30) and from the damping means (30) onto the lower support (20) forward onto the heel of the person or the heel of the shoe of the person.

4. The posture assisting device according to any of claims 1 to 3, **characterized in that** said damping means (30) comprises a liquid-type damper or a viscous type damper means and that said control means includes a valve.

5. The posture assisting device according to any of claims 1 to 4, **characterized in that** said control means additionally allow at least one state (102) having a lower movement than said drive state (103).

6. The posture assisting device according to any of claims 1 to 5, **characterized in that** said control means additionally allow to continuously control the movement between said locking state (101) and said drive state (103).

7. The posture assisting device according to any of claims 4 to 6, **characterized in that** said control means comprises a manually driven lever (33) to control said valve.

8. The posture assisting device according to any of claims 4 to 7, **characterized in that** said control means comprises an electrically driven valve.

9. The posture assisting device according to any of claims 1 to 8, **characterized by** laces for connecting the upper support (10) to the thigh of the person and/or for connecting the lower support (20) to the shank of said person, preferably by means of hook-and-loop fasteners.

10. The posture assisting device according to any of claims 1 to 9, **characterized in that** the upper support (10) and/or the lower support (20) are adjustable in
respect of the length as to fit comfortably to persons with different length of the thigh and the shank, respectively.
